# EUROPEAN PATENT SPECIFICATION

(11) **EP 0 787 496 B1**
(45) Date of publication and mention of the grant of the patent: **28.05.2003**
(21) Application number: 96830038.4
(22) Date of filing: 31.01.1996
(51) Int. Cl.: A61K 35/78

(54) **Process through which to obtain multifraction dry extracts from plants**
Verfahren zur Gewinnung von Multifraktions-Trockenextrakten aus Pflanzen
Procédé d'obtention de fractions multiples d'extraits secs de plantes

(43) Date of publication of application: 06.08.1997
(73) Proprietor: ABOCA S.p.A., 52037 Sansepolcro (Arezzo) (IT)
(72) Inventor: Mercati, Valentino, 06012 Citta'Di Castello, Perugia (IT)
(74) Representative: Berneschi, Ciro

(56) References cited:
- IT-A- 1 190 134
- US-A- 5 051 258
- DATABASE WPI Section Ch, Week 8238 Derwent Publications Ltd., London, GB; Class B04, AN 82-80005E XP002008770 & JP-A-57 130 920 (ZENYAKU KOGYO KK) , 13 August 1982
- DATABASE WPI Section Ch, Week 9440 Derwent Publications Ltd., London, GB; Class B04, AN 94-322105 XP002008771 & JP-A-06 247 867 (AKIYAMA SANGYO KK) , 6 September 1994
- DATABASE WPI Section Ch, Week 8501 Derwent Publications Ltd., London, GB; Class B04, AN 85-004171 XP002008772 & JP-A-59 205 324 (OHYA K) , 20 November 1984
- DATABASE WPI Section Ch, Week 8226 Derwent Publications Ltd., London, GB; Class B04, AN 82-53658E XP002008773 & JP-A-57 082 319 (GEN K) , 22 May 1982
- DATABASE WPI Section Ch, Week 8009 Derwent Publications Ltd., London, GB; Class B03, AN 80-15340C XP002008774 & JP-A-55 007 039 (MASUYAMA F) , 18 January 1980

## Description

The present invention relates to a process through which to obtain dry extracts usable in pharmaceutical, herbal, cosmetic and dietetic fields, with a high titre of active principles representative of the phytocomplex and with suitable physico-chemical characteristics being suitable for commercialization.

In the field of medicinal and aromatic plants the most important problem to solve is to obtain end products with a high content of active principles, in order for these products to carry out the desired pharmacological, dietetic or cosmetic actions.

According to this the main parameters of the end products are two: the concentration of the active principles, and the physico-chemical characteristics, as for example the specific weight of the powder that will be used to produce capsules, tablets, granules, ect.

To reach said aim the vegetable substances are treated to obtain dry extracts at high concentration of active principles.

The dry extracts that are obtained through existing processes can not be generally used as such because they are not workable and they must be added with inert excipients (usually at the rate of 15%-30%), which inevitably cause a lowering of the active principles' titre reached through the extraction.

In order to limit this inconvenience a possible solution is to obtain more and more concentrated extracts, for example by improving the choice of solvents and/or in case by fractionating the whole extract in order to obtain one or more isolated active principles.

However, with this method, we obtain an end product that contains only a part of the phytocomplex or at least only one of the substances to which the activity is attributed, while the other secondary metabolites are lost. In most of the cases this represents a problem either because the substance causing the wanted action is not known or because it is known that this action is carried out from the whole of the active principles.

Some of these limitations and inconveniences have been overcome by the invention matter of the Italian Patent 1190134 of June 19, 1986 filed in the name of the same firm ABOCA by the same inventor Mercati Valentino.

Such process, thanks to the use of the residual powdered vegetable product, allows to obtain a workable dry extract without the addition of inert excipients that would inevitably cause the lowering of the concentration of the active principles.

Furthermore, the addition of the residual vegetable material allows to reconstruct the drug phytocomplex, that is to obtain a product that, besides the extracted fraction of active principles, also contains the other secondary metabolites existing in the plant.

However, said process had the limitation of being able to concentrate only a fraction of the plant's active principles, namely the fraction extracted from the chosen solvent, and to use only the residual vegetable and not the integral one.

The aim of this invention is to find a process through which a plant can be treated so as to allow:
1) the extraction of the greatest possible quantity of substances through a plurality of different solvents to allow the separation of said substances at least in separate groups;
2) the obtaining of an end phytocomplex in which the contents of active principles can be regulated both qualitatively and quantitatively;
3) the obtaining of a workable dry extract without the addition of inert excipients or with a minimum addition of them, so that said excipients do not affect in a significant way the whole content of the active principles;
4) the obtaining of a dry extract with physico-chemical characteristics being suitable for commercialization, for example with a high specific weight;
5) the fractioned recover of at least high percentages of distinct solvents in order to allow their reuse, and therefore the production of minimum quantities of mixtures of different solvents to be destroyed.

The invention that allows to obtain such results relates to a process consisting in a succession of separate interaction phases, with solvents: the first phase concerns the action of a first solvent on the vegetable product to treat, action carried out in temperature conditions compatible with the stability of the active principles, for a duration in hours and with a plant-solvent weight ratio that are suitable, in order to extract an active principle or a group of active principles; afterwards, after having taken out the first solution, the residual vegetable undergoes the action of a second solvent, then eventually of an nth one, at the temperature, duration and plant-solvent weight ratio that are adequate and suitable for each phase, in order to extract, with each solvent, another active principle or another group of active principles.

So at the end of the cycle we obtain the residual vegetable on one side and n distinct solutions on the other side, each solution having inside one or more dissolved active principles.

Such solutions can be according to the cases:
1) mixed as they are in pre-determined proportions of the weight contents of the extracts and then the resulting mixture will be concentrated;
2) separately concentrated to recover the single solvents and then mixed in pre-determined proportions of the weight contents of the extracts.

The obtained concentrated mixture is dried, if necessary through lyophilization, in order to obtain, in both cases, a multifraction dry extract with the desired content of active principles and according to the cases added or not of the integral powdered vegetable and/or of the residual powdered vegetable, and in case added of minimum quantities of inert excipients.

Such process reveals itself to be particularly advantageous because the actions of the single solvents are regulated for each of them by: an extraction temperature compatible with the stability of the active principles, an extraction duration and by a plant-solvent weight ratio that allow to obtain the maximum extraction of the different fractions of the plant's active principles that depend on the type of vegetable, on the type of solvent and on the extraction phases that the plant has already undergone in the same process.

Moreover, the right choice of solvents and of extractive parameters (temperature, duration, plant-solvent weight ratio), as well as of the optimum mixing ratio of the obtained extracts allow to create a product with the wanted content of active principles both qualitatively and quantitatively, and workable without the addition of inert excipients, or with a minimum addition of them so that they do not affect, in a significant way, the whole content of the active principles.

Finally, the right choice of concentration of the fluid extracts to dry, if necessary through lyophilization, makes it possible to obtain a multifraction dry extract, with high, or anyway optimum, specific weight of the end product for the commercialization in the chosen dosage form.

Further characteristics of the invention and the advantages it allows to reach will appear evident thanks to the following description and to the block diagrams in the enclosed drawing, all given as a mere example of the object of this invention, and they are by no means intended to restrict it, and in which:
- figure 1 is the block description of the succession of phases of the general process;
- figure 2 is the block description of the succession of phases concerning the treatment of Passiflora incarnata.

In the graphic description letter A indicates the vegetable product to treat, B indicates the phase in which the vegetable product is triturated so as to facilitate the action of the solvents in the following extraction phases, C indicates the first phase in which -usually inside a solvent recycling percolator or in equivalent apparatuses- the interaction between the first solvent S1 and the triturated plant takes place, at a temperature, a duration and with a plant-solvent weight ratio that are determined.

The interaction phase between the vegetable product and the solvent is followed by a separation phase with which the liquid phase is gathered in L, where said liquid phase can undergo a concentration process after which a relevant part of the solvent S1 is gathered in S1, said solvent S1 can thus be reused, while the first extract in the shape of a fluid extract with a high percentage of active principles is gathered in M.

The remaining vegetable product undergoes the action of n solvents S2 ....Sn in following phases at the temperature, duration and plant-solvent weight ratio that are determined according to the used solvents and to the active principles to extract from the vegetable.

At the end of the nth extraction phase, the liquid phase is gathered in container N, the residual vegetable in container E, where it is usually dried in order to be pulverized or it is sent to the authorized dump.

The liquid phase in N can undergo a concentration with separation to one side of solvent Sn, which can then be reused, on the other side (in P) of the fluid extract at high concentration of active principles that are extracted from the nth Sn solvent.

The products obtained in the above-mentioned intermediate phases and gathered in the containers, from M to P, all of said products or the ones desired are mixed together according to the pre-determined optimum proportions in mixer F, at which exit we have a homogeneous product that is gathered in concentrator G, recovering the solvents in R. Alternatively, the extracts from L to N, all of them or the desired ones, can be mixed as they are in the optimum proportions and thus concentrated in G phase recovering the solvents still in R.

In both cases, after the G phase, we obtain, in H, a compound which is still fluid enough to be dried, if necessary through lyophilization, and then transformed in dry powder I, said compound can be added or not of a given quantity of powdered integral vegetable and/or of powdered residual vegetable.

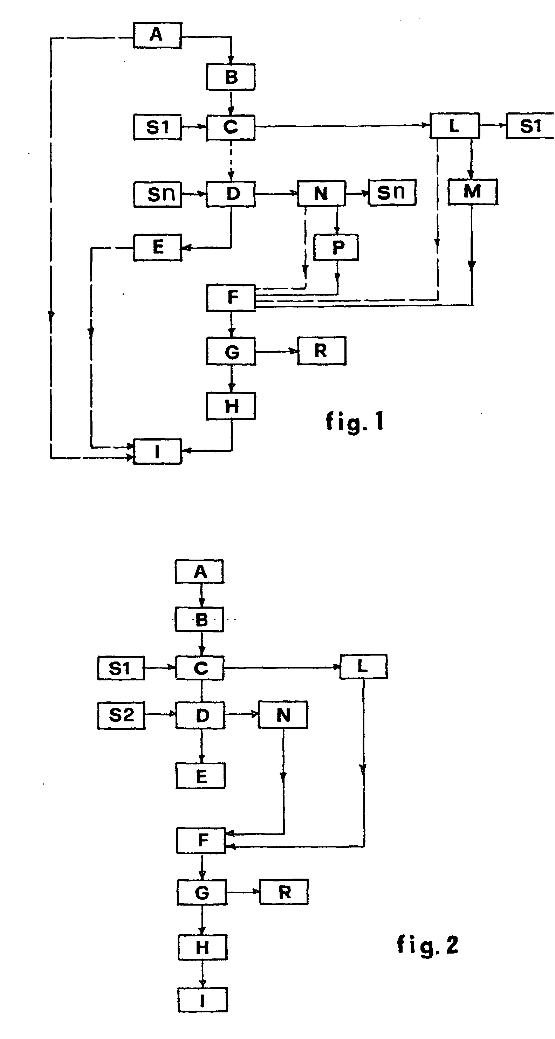

The diagram of figure 2 shows a practical example of accomplishment of the invention referred to the treatment of the Passiflora incarnata, a well known medicinal plant, from which to extract a phytocomplex endowed with pharmacological action, e.g. sedative, antiinflammatory and spasmolytic activities.

In this case the used solvents are two: the first S1 is ethanol at about 40° and it is forced to act inside the apparatus or percolator C at a processing temperature ranging from 45° to 50° C for a duration ranging from 3 to 6 hours, after maceration from 12 to 15 hours at room temperature, with a plant-solvent weight ratio ranging from 1: 10 to 1: 20. In such a phase the medium-polar fraction, gathered in L, is extracted from the Passiflora incarnata.

In percolator D that can be the same one indicated with C, after the preceding liquid phase has been removed from the same percolator- the second solvent S2 is demineralized water at 60°-80° C. The extraction time ranges from 8 to 24 hours and the plant-solvent weight ratio still ranges from 1: 10 to 1: 20. In this second phase the high-polar fraction is extracted in N from the residue of the Passiflora incarnata.

In the exemplified process the two fluid extracts L and N are mixed in F according to proportions that safisfy the weight proportions of the desired active principles. In particular the weight percentage of the dry residue coming from the water solution usually ranges from 10% to 30% of the totality of the lyophilized end extract, so to render the product workable at standardized titre. The so obtained mixture is concentrated in G for the elimination of alcohol and of a part of water, both to gather in R, which can be reused for further extractions; such a phase allows to obtain an optimum concentration at the entry of the lyophilizator so as to have, in exit I, the end product with physico-chemical characteristics (for example specific weight) being suitable for commercialization in an adequate pharmaceutical dosage form without the use of inert excipients.

As an example, the evaporation of the solvents gathered in G, before the lyophilization, is conducted till obtaining a concentration of about 300-500 mg/ml. The specific weight of the dry residue, obtained after lyophilization, appears to be approx. 0.7 mg/ml, such specific weight allows to prepare capsules of 0.5 ml containing up to about 360 mg of dry extract.

The high solubility in water (about 500 mg/ml) of the dry residue also allows a rapid absorption of the active principles contained in the pharmaceutical dosage form given orally, already at the gastrointestinal tract. In this case the right choice of solvents (ethanol at 40° and demineralized water) and of other parameters that affect the extractions, as well as of the proportions according to which make the mixture, brings to the obtaining of a standardizable dried and workable extract without having to add inert excipients or the powdered plant or the residual vegetable. Furthermore, such dry extract contains two extractive fractions with the highest amount of all the flavonoids existing in the plant, both the most and the least polar ones.

As an example, the content of the flavonoids on the total of the dry residue, expressed as addition of the weights of the single flavonoids, is of about 9%, with a remarkable increase with respect to the titre of the initial plant (about 3,3%). In the ethanolic extract the content of flavonoids on the total of the dry residue reveals to be of about 10% and in the water extract of about 3,5%, of which (about) 60% is made of the most polar flavonoids and (about) 40% is made of the least polar flavonoids.

## Claims

1. A process through which to obtain multifraction dry extracts from plants, particularly from the medicinal ones, with which to create products usable in pharmaceutical, herbal, cosmetic and dietetic fields, rich in active principles representative of the phytocomplex and with suitable physico-chemical characteristics for commercialization, said process comprising a preparation phase of the plant in which it is finely chopped up, phases in which the plant interacts with solvents for the extraction of the active principles contained in said plant, as well as phases with which the mixture of solutions containing the active principles is concentrated in order to be finally reduced to a dry pulverulent state -if necessary through lyophilization-, said process being **characterized by**:
- a succession of separate extraction phases, in each of such phases the plant interacts with a distinct solvent, in temperature conditions adequate for the stability of the extracted active principles and for a duration and a plant-solvent weight ratio adequate for the active principles or for the group of active principles to be extracted, with which to extract an active principle or a group of active principles, after which the so obtained distinct solutions are gathered in different containers;
- a phase in which at least some of the single extractive solutions are first mixed according to pre-determined proportions of the weight contents of the extracts and then the so obtained mixture will be concentrated, or a phase in which at least some of the single extractive solutions are separately concentrated and then mixed according to pre-determined proportions of the weight contents of the extracts, in order to obtain a multifraction dry extract with the desidered content of a active principles, both qualitatively and quantitatively;
- a phase in which, after the succession of extractions, the remaining vegetable product is dried, in one case to be pulverized and then a portion is added to the multifraction dry extract, in another case to be gathered and sent to the authorized dump.

2. A process through which to obtain multifraction dry extracts from plants, particularly from the medicinal ones, with which to create products usable in pharmaceutical, herbal, cosmetic and dietetic fields, rich in active principles representative of the phytocomplex and with suitable physico-chemical characteristics for commercialization, said process for the elaboration of the Passiflora incarnata, comprising a preparation phase of the plant in which it is finely chopped up, as well as two distinct phases for the extraction of the active principles contained in said plant, as well as phases with which the mixture of solutions containing the active principles is concentrated in order to be finally reduced to a dry pulverulent state -if necessary through lyophilization-, **characterized by** the fact that in the first phase the extraction is carried out with ethanol at about 40°, processing temperature ranging from 45° to 50° C, duration from 3 to 6 hours and plant-solvent weight ratio ranging from 1: 10 to 1: 20 for the extraction of the medium-polar fraction, that the second extraction phase is carried out with demineralized water at a temperature ranging from 60° to 80° C, duration ranging from 8 to 24 hours, plant-solvent weight ratio from about 1: 10 to 1: 20 for the extraction of the high-polar fraction, and **characterized by** the fact that the mixing of the two extracts is carried out so that the weight percentage of the dry residue coming from the water solution ranges from 10% to 30% of the totality of the lyophilized extract, so to render the product workable at standardized titre, and such as that the solution concentration before the lyophilization is about 300-500 mg/ml.

## Patentansprüche

1. Verfahren zur Gewinnung von Multifraktions-Trockenextrakten aus Pflanzen, insbesondere aus Arzneipflanzen, zur Herstellung von Produkten, die auf pharmazeutischem, pflanzlichem, kosmetischem und diätetischem Gebiet brauchbar sind, reich an repräsentativen Wirkstoffen des Phytokomplexes sind und geeignete physikalisch-chemische Eigenschaften für eine Kommerzialisierung haben, wobei das Verfahren eine Aufbereitungsphase, in der die Pflanze fein zerkleinert wird, Phasen, in denen die Pflanze mit Lösungsmitteln zur Extraktion der in der Pflanze enthaltenen wirksamen Bestandteile behandelt wird und Phasen umfasst, in denen das die wirksamen Bestandteile enthaltende Gemisch der Lösungen konzentriert wird, um schließlich - nötigenfalls durch Gefriertrocknung - in ein trockenes Pulver überführt zu werden,
**gekennzeichnet durch**:
eine Folge von getrennten Extraktionsphasen zur Extraktion eines wirksamen Bestandteils oder einer Gruppe wirksamer Bestandteile, wobei in jeder Phase die Pflanze mit einem anderen Lösungsmittel bei einer der Stabilität der zu extrahierenden wirksamen Bestandteile angemessenen Temperatur und bei einer dem zu extrahierenden wirksamen Bestandteil oder der Gruppe der zu extrahierenden wirksamen Bestandteile angemessenen Dauer und Gewichtsverhältnis von Pflanze zu Lösungsmittel behandelt wird und anschließend die erhaltenen getrennten Lösungen in verschiedenen Behältern gesammelt werden;
eine Phase, in der wenigstens einige der einzelnen Extraktlösungen in bestimmten Proportionen der Gewichtsgehalte der Extrakte zunächst gemischt werden und das erhaltene Gemisch anschließend konzentriert wird oder eine Phase, in der wenigstens einige der einzelnen Extraktlösungen getrennt konzentriert und danach in bestimmten Proportionen der Gewichtsgehalte der Extrakte gemischt werden, so dass man einen Multifraktions-Trockenextrakt mit dem - sowohl qualitativ wie auch quantitativ - gewünschten Gehalt an wirksamen Bestandteilen erhält;
eine Phase, in der nach den Extraktionsfolgen das verbliebene pflanzliche Produkt getrocknet und entweder pulverisiert wird und anschließend ein Anteil dem Multifraktions-Trockenextrakt zugegeben wird oder gesammelt und deponiert wird.

2. Verfahren zur Gewinnung von Multifraktions-Trockenextrakten aus Pflanzen, insbesondere aus Arzneipflanzen, zur Herstellung von Produkten, die auf pharmazeutischem, pflanzlichem, kosmetischem und diätetischem Gebiet brauchbar sind, reich an repräsentativen Wirkstoffen des Phytokomplexes sind und geeignete physikalisch-chemische Eigenschaften für eine Kommerzialisierung haben, wobei das Verfahren der Verarbeitung von *Passiflora incarnata* dient und eine Aufbereitungsphase, in der die Pflanze fein zerkleinert wird und zwei getrennte Phasen zur Extraktion der in der Pflanze enthaltenen wirksamen Bestandteile sowie Phasen umfasst, in denen das die wirksamen Bestandteile enthaltende Gemisch der Lösungen konzentriert wird, um schließlich - nötigenfalls durch Gefriertrocknung - in ein trockenes Pulver überführt zu werden, **dadurch gekennzeichnet, dass**
- in der ersten Phase die Extraktion mit Ethanol bei etwa 40 ° durchgeführt wird, die Verarbeitungstemperatur im Bereich von 45 °C bis 50 °C liegt, die Behandlung 3 bis 6 Stunden dauert und das Gewichtsverhältnis von Pflanze zu Lösungsmittel im Bereich von 1:10 bis 1:20 liegt zur Extraktion einer Fraktion mittlerer Polarität, dass die zweite Extraktionsphase mit entmineralisiertem Wasser 8 bis 24 Stunden bei einer Temperatur im Bereich von 60 °C bis 80 °C und einem Gewichtsverhältnis von Pflanze zu Lösungsmittel im Bereich von 1:10 bis 1:20 durchgeführt wird zur Extraktion der hochpolaren Fraktion,
- und **dadurch gekennzeichnet, dass** das Mischen der zwei Extrakte so erfolgt, dass der Gewichtsprozentsatz des trockenen, aus der wässrigen Lösung stammenden Rückstandes im Bereich von 10 % bis 30 % des gesamten gefriergetrockneten Extraktes liegt, um das Produkt auf einen bestimmten Titer zu standardisieren und die Konzentration der Lösung vor der Gefriertrocknung etwa 300-500 mg/ml beträgt.

## Revendications

1. Un procédé permettant d'obtenir des extraits secs multifractions de plantes, en particulier de plantes médicinales, avec lesquels on peut créer des produits utilisables dans les domaines pharmaceutique, cosmétique, diététique et de l'herboristerie, riches en principes actifs représentatifs du phytocomplexe et avec des caractéristiques physico-chimiques appropriées pour une commercialisation, ledit procédé comprenant une phase de préparation de la plante, dans laquelle elle est finement coupée en morceaux, des phases dans lesquelles la plante interagit avec des solvants pour l'extraction des principes actifs contenus dans ladite plante, ainsi que des phases avec lesquelles le mélange de solutions contenant les principes actifs est concentré afin d'être finalement réduit à un état pulvérulent sec, si nécessaire par lyophilisation, ledit procédé étant **caractérisé par** :
- une succession de phases d'extraction, dans chacune de ces phases, la plante interagit avec un solvant différent, dans des conditions de température adéquates pour la stabilité des principes actifs extraits, et pour une durée et une proportion pondérale plante-solvant appropriées pour le principe actif ou le groupe de principes actifs à extraire, avec lesquelles on extrait un principe actif ou un groupe de principes actifs, après quoi, les différentes solutions ainsi obtenues sont recueillies dans différents récipients ;
- une phase dans laquelle au moins certaines des solutions extractives simples sont tout d'abord mélangées selon des proportions prédéterminées de teneur en poids des extraits, puis, le mélange ainsi obtenu sera concentré, ou une phase dans laquelle au moins certaines des solutions extractives simples sont concentrées séparément, puis mélangées selon des proportions prédéterminées de teneur en poids des extraits, afin d'obtenir un extrait sec multifraction avec la teneur désirée d'un principe actif, de manière à la fois qualitative et quantitative ;
- une phase dans laquelle, après la succession d'extractions, le produit végétale restant est séché, dans un cas pour être pulvérisé, puis une portion est ajoutée à l'extrait sec multifraction, dans un autre cas, pour être recueilli et envoyé vers la décharge agréée.

2. Un procédé permettant d'obtenir des extraits secs multifractions de plantes, en particulier de plantes médicinales, avec lesquels on peut créer des produits utilisables dans les domaines pharmaceutique, cosmétique, diététique et de l'herboristerie, riches en principes actifs représentatifs du phytocomplexe et avec des caractéristiques physico-chimiques appropriées pour une commercialisation, ledit procédé pour l'élaboration de la Passiflora incarnata comprenant une phase de préparation de la plante, dans laquelle elle est finement coupée en morceaux, ainsi que deux phases distinctes pour l'extraction des principes actifs contenus dans ladite plante, ainsi que des phases avec lesquelles le mélange de solutions contenant les principes actifs est concentré afin d'être finalement réduit à un état pulvérulent sec, si nécessaire par lyophilisation, **caractérisé par le fait que** dans la première phase, l'extraction est mise en oeuvre avec de l'éthanol à environ 40°, une température de traitement allant de 45 ° à 50 °C, une durée de 3 à 6 heures et une proportion pondérale plante-solvant allant de 1:10 à 1:20 pour l'extraction de la fraction moyennement polaire, que la deuxième phase d'extraction est mise en oeuvre avec de l'eau déminéralisée à une température allant de 60 ° à 80 °C, une durée allant de 8 à 24 heures, une proportion pondérale plante-solvant d'environ 1:10 à 1:20 pour l'extraction de la fraction hautement polaire, et **caractérisé par le fait que** le mélange des deux extraits est effectué de telle manière que le pourcentage en poids du résidu sec provenant de la solution aqueuse se situe entre 10 % et 30 % de la totalité de l'extrait lyophilisé, de façon à rendre le produit réalisable à un titre normalisé et de telle sorte que la concentration de la solution avant lyophilisation est d'environ 300-500 mg/ml.
